# FASCICULE DE BREVET EUROPEEN

(11) **EP 0 304 369 B1**
(45) Date de publication et mention de la délivrance du brevet: **12.04.1995**
(21) Numéro de dépôt: 88402049.6
(22) Date de dépôt: 05.08.1988
(51) Int. Cl.: C07F 5/00

(54) **Procédé de préparation de dispersions colloidales de dioxyde cérique en milieu alcoolique**
Verfahren zur Herstellung von kolloidalen Dispersionen von Cerdioxid in alkoholischem Medium
Process for the preparation of colloidal dispersions of ceric dioxide in an alcoholic medium

(30) Priorité: 19.08.1987 US 87076
(43) Date de publication de la demande: 22.02.1989
(73) Titulaire: RHONE-POULENC CHIMIE, 92408 Courbevoie Cédex (FR)
(72) Inventeur: Gradeff, Peter S., Pottersville New Jersey 08979 (US); Ramirez, Carlos, Piscataway New Jersey 08854 (US)
(74) Mandataire: Dutruc-Rosset, Marie-Claude

(56) Documents cités:
- EP-A- 0 097 563
- US-A- 3 231 592

## Description

La présente invention a pour objet un nouveau procédé de préparation de dispersions colloïdales de dioxyde cérique en milieu alcoolique.

Il est bien connu que les savons métalliques sont utilisés comme siccatifs dans les formulations de peintures et vernis, en vue d'accélérer le séchage des huiles insaturées telles que l'huile de lin ou les résines synthétiques insaturées telles que les résines alkydes. On pense que le cation du savon métallique catalyse activement les processus d'oxydation et de polymérisation et que l'anion de l'acide sert de support au métal conférant ainsi la solubilité dans l'huile, l'insolubilité dans l'eau et la comptabilité avec les autres composants de la peinture.

Il ressort du brevet GB 1 236 085 que, de toute évidence, il est avantageux d'un point de vue économique d'incorporer autant de métal que possible par unité d'acide à condition que le savon obtenu soit soluble dans l'huile. On y parvient en mettant en oeuvre des savons "basiques" dans lesquels le rapport du métal à l'acide est supérieur au rapport stoechiométrique, par exemple :
2 R COOH + PbO → (R COO)₂ Pb + H₂O - rapport stoechiométrique
2 R COOH + 2 PbO → R COO Pb.O.Pb OOC.R + H₂O- savon "basique"
Cependant, le brevet indique que la solution de savon et d'huile que l'on obtient, lors de la préparation des savons "basiques" de ce type, est tellement visqueuse qu'elle est difficile à manipuler, en particulier au cours des opérations de mélange nécessaires durant la fabrication des compositions de peinture. Selon le brevet britannique, on peut réduire cette viscosité élevée en faisant réagir le milieu réactionnel de l'acide carboxylique ou de son sel de métal alcalin avec un sel de métal polyvalent ou un oxyde métallique apportant le cation métallique du siccatif.

Le sel de métal polyvalent ou l'oxyde métallique utilisé dans ledit procédé est un sel ou un oxyde d'aluminium, de baryum, de cuivre, de fer ou de mangésium ; de préférence de zirconium, de zinc ou de manganèse ; et encore plus préférentiellement de calcium, de plomb ou de cobalt. Il est recommandé de mélanger les différents savons métalliques, d'autant plus que certains savons tels que les savons de zinc et de calcium n'agissent pas eux-mêmes comme siccatifs, mais exercent un effet de synergie sur d'autres savons tels que les savons de cobalt ou de plomb. Il n'est pas fait référence aux savons de cérium ou des métaux de terres rares.

Le brevet GB 972 804 décrit des savons organiques de métaux contenant de l'aluminium ou du bore et au moins un métal divalent ou un radical métallique divalent (les atomes d'aluminium ou de bore et du métal divalent étant reliés par des atomes d'oxygène) et au moins un radical d'acide carboxylique. On obtient ces composés organiques de métaux par condensation des alcoxydes ou aryloxydes d'aluminium ou de bore avec des acyloxydes de métaux ou de radicaux métalliques divalents. Les métaux et les radicaux métalliques divalents comprennent le magnésium, le calcium, le strontium, le baryum, le zinc, le cadmium, le fer, le cobalt, le nickel, le plomb, le cuivre, le manganèse et le radical zirconyle, mais il n'est pas fait référence aux métaux ou radicaux des terres rares tel que le cérium. Les produits ont une teneur élevée en métal, les radicaux acides organiques étant présents dans la proportion de 0,5 à 1,5 équivalents par atome de métal. Il en résulte que les produits ont un potentiel d'acceptation des acides supérieur à celui des savons métalliques classiques. Ceux-ci constituent donc un exemple du type de savons "basiques" dont il est question dans le brevet GB 1 236 085 précité.

Il est mentionné dans le brevet GB 1 430 347 de Collins et Pearl que les savons métalliques normaux ou basiques d'acides carboxyliques synthétiques étaient des composés analogues à ceux qui dérivaient antérieurement d'acides naturels, et qu'ils ont renfermé des composés ayant entre eux une relation plus ou moins homologue sinon isomère lorsque l'on a utilisé les différents acides carboxyliques synthétiques au fur-et-à-mesure de leur disponibilité. Collins et al. proposent, à partir de cet art antérieur, de mettre en oeuvre une méthode de préparation différente et une composition différente ce qui a pour résultat de conférer au produit siccatif ou savon métallique obtenu un caractère et des propriétés différents.

Dans l'art antérieur, selon Collins et al, les savons métalliques ont été préparés par des procédés de fusion ou de précipitation. L'acide qui va réagir peut être dissous dans un solvant inerte approprié, généralement un solvant hydrocarboné, tel qu'une essence minérale, auquel on ajoute ensuite le composé métallique désiré, généralement sous forme d'un oxyde, d'un composé inorganique ou d'un sel approprié, en chauffant à une température appropriée pour favoriser la réaction. On obtient ainsi une solution hydrocarbonée de ce savon et le solvant peut être éliminé par distillation pour augmenter la concentration en métal à la valeur souhaitée.

Le procédé Collins et al. consiste à mettre en oeuvre un acide carboxylique ou un mélange d'acides qui peuvent être d'origine naturelle, dérivés d'un produit naturel ou d'origine synthétique et à le mélanger avec un glycol-éther, un glycol ou autre polyol analogue, tout en ajoutant également un composé métallique tel que le métal sous forme de poudre ou un oxyde, hydroxyde, acétate ou carbonate dudit métal. On chauffe alors ce mélange à une température allant de 65° à 143°C jusqu'à ce que le composé métallique disparaisse. Ensuite, on élimine l'eau par distillation, on filtre le mélange réactionnel et on élimine l'excès de glycol ou de glycol-éther par distillation jusqu'à obtention de la concentration ou de l'état convenable souhaité.

Le rapport des équivalents de métal au glycol-éther ou polyol est d'au moins 0,5, mais il faut conserver une quantité significative de glycol-éther ou de polyol dans le produit si l'on souhaite qu'il reste fluide. Le rapport des équivalents de métal à l'acide est d'au moins 1,0 et lorsque le métal est le plomb, il est d'au moins 1,5 ; avec le plomb on obtient facilement des rapports de 2 et plus. En dehors du plomb, on a préparé également selon ce procédé des savons de baryum, de nickel et de manganèse ainsi que des savons de cobalt. Cependant, on n'y trouve aucune référence aux métaux de terres rares tels que le cérium.

Il est précisé dans ce brevet que le produit et le procédé sont nettement différents des propositions de l'art antérieur, par exemple, l'emploi de quantités variables de glycol ou de glycol-éther simplement pour réduire la viscosité du carboxylate de plomb comme dans le brevet GB n° 1 148 998 ou pour stabiliser des solutions de savon comme dans le brevet US n° 2 807 553 de Fisher. Ces produits sont commercialisés par la Société Mooney Chemicals.

La demande de brevet FR 76 22426 publiée sous le numéro 2 359 192 et le brevet GB 1 571 210 concernent des sels organiques de cérium solubles dans les solvants organiques, caractérisés par un rapport r du nombre d'équivalents d'acide au nombre d'atomes de cérium compris entre 0,2 et 1 ; le nombre d'équivalents d'acide représente le nombre de molécules d'acide lorsque l'acide utilisé est monofonctionnel, et il faut doubler ou tripler ce nombre, dans le cas de diacides ou triacides et, plus généralement, le multiplier par le nombre de fonctions acide dans le cas d'un polyacide. Les composés du cérium ainsi proposés nécessitent une quantité d'acide beaucoup plus faible que la quantité utilisée antérieurement pour obtenir la même efficacité ; on peut également obtenir des solutions de concentration élevée en métal pouvant aller jusqu'à 500 g/l ; les solutions obtenues restent fluides et peuvent être manipulées sans aucune difficulté tout en restant parfaitement solubles dans les milieux d'hydrocarbures.

L'acide organique peut être RC OOH, R SO₃H, RO SO₃H, RO PO₃ H₂ ou (RO)₂ PO₂H, R étant un radical hydrocarboné ayant au moins 7 atomes de carbone. Le radical acide organique peut être un radical aliphatique linéaire ou ramifié ou un radical cycloaliphatique éventuellement substitué par un radical alcoyle ou un radical aromatique éventuellement substitué par un radical alcoyle. Les sels de cérium de ces acides organiques peuvent, en plus, contenir au moins un autre métal de terres rares et ce, jusqu'à 25 % de la teneur totale en métaux de terres rares, y compris le cérium. On peut obtenir des compositions fournies sous la forme de solution dans un solvant organique, d'un sel de cérium d'acide organique ou d'un mélange de sels contenant plus de 200 g de cérium par litre de composition. Ces compositions peuvent être ajoutées aux peintures, vernis ou combustibles liquides.

Le procédé de préparation de ces sels de cérium d'acide organique ou de leurs mélanges consiste à faire réagir dans un milieu organique ou hydroorganique, l'acide organique et l'hydroxyde de cérium Ce (OH)₃ fraichement préparé de sorte que les sels de cérium d'acide organique obtenus aient un rapport r compris entre 0,2 et 1. La réaction s'effectue de préférence en chauffant et le milieu organique est de préférence un hydrocarbure. Après plusieurs heures, une partie de l'eau qui s'est formée durant la réaction décante spontanément. Aprés réaction, il est possible d'aider à la séparation de l'eau formée à partir du milieu réactionnel en ajoutant un tiers solvant tel qu'un glycol, un alcool ou un alcoylglycol. On peut ajuster la concentration de la solution ainsi obtenue en ajoutant un hydrocarbure approprié.

Dans les exemples, l'hydroxyde de cérium Ce (OH)₃ est obtenu par précipitation du nitrate de cérium par l'ammoniaque. On lave le précipité à l'eau jusqu'à disparition de l'ion nitrate ; ensuite, on filtre le précipité jusqu'à ce qu'il ne contienne plus que 15 % d'eau. On fait réagir à 80°C l'hydroxyde de cérium avec 130 g d'acide oléique technique dans du white-spirit. Après quatre heures on ajoute du glycol, on élimine l'eau qui s'est séparée et, ensuite, on ajoute du butyl-glycol, puis du white-spirit pour constituer la solution finale.

La demande de brevet FR 81 09214 publiée sous le numéro 2 482 075 et le brevet US 4 356 106 sont relatifs à la préparation de dispersions aqueuses de composés du cérium qui peuvent être aisément dispersés. On obtient un produit dispersable en milieu aqueux en chauffant l'hydrate de dioxyde de cérium contenant des ions NO₃⁻, Cl⁻ ou ClO₄⁻, pendant 1 à 2 heures, à des températures comprises entre 200 et 450°C. Cependant, on ne donne aucune indication sur l'aptitude du produit à se disperser en milieu organique.

On notera que l'invention du brevet se rapporte aux sels céreux et non pas aux sels cériques.

Dans l'encyclopédie Kirk-Othmer, Encyclopedia of Chemical Technology (deuxième édition), volume 4, page 850, il est indiqué que l'oxyde cérique hydraté, également dénommé "hydrous ceric oxide" ou "cerium hydroxide" CeO₂ x H₂O, formule dans laquelle x est un nombre compris entre 1/2 et 2, est obtenu sous forme d'un précipité gélatineux lorsqu'on ajoute des hydroxydes de sodium ou d'ammonium aux solutions de sels cériques. Après séchage du précipité, on obtient un oxyde hydraté jaune contenant de 85 à 90 % de CeO₂. On peut obtenir de l'hydroxyde cérique granulaire en faisant bouillir des sels de cérium insolubles avec de l'hydroxyde de sodium concentré et en procédant ensuite à un lavage et un séchage. La composition et la structure de ces composés dépendent de leur mode de préparation ; dans de nombreux cas, elles sont incertaines. C'est la raison pour laquelle, dans la pratique, on exprime la composition en termes d'équivalent CeO₂.

L'hydroxyde céreux Ce(OH)₃ est obtenu sous forme d'un précipité gélatineux blanc ou blanchâtre lorsqu'on alcalinise des solutions contenant l'ion céreux Ce³⁺. Quand on laisse reposer un certain temps, il apparaît une couche superficielle d'hydroxyde céreux/cérique.

On obtient généralement l'oxyde cérique CeO₂ par calcination à l'air de l'oxalate céreux, de l'hydroxyde céreux ou cérique. L'oxyde cérique est insoluble dans les acides, mais sa dissolution est accélérée par l'addition d'une petite quantité d'agent réducteur tel qu'un iodure ou l'eau oxygénée. Eventuellement, les acides nitrique ou sulfurique concentrés réagissent à chaud.

Dans de nombreuses applications, on peut remplacer l'oxyde cérique par l'oxyde cérique hydraté. Cependant, à la différence de l'hydroxyde céreux qui est un type classique d'hydroxyde métallique analogue au Pb(OH)₂, au Fe(OH)₃ etc..., l'hydroxyde cérique est, en fait, du dioxyde cérique hydraté. Dans l'exposé qui suit de l'invention, on entend par "dioxyde cérique" aussi bien le dioxyde cérique, l'hydroxyde cérique, le dioxyde cérique hydraté (quelle que soit la forme sous laquelle l'eau est retenue dans ou sur le produit : eau libre, eau liée ou adsorbée, eau de cristallisation).

Si l'on agite et chauffe du dioxyde cérique pur à une température comprise entre 60° et 200°C en présence d'un solvant aliphatique, tel que l'éther de pétrole, ou d'un solvant aromatique, tel que le toluène, et en présence d'un acide carboxylique, tel que l'acide oléique ou palmitique ou dodécylbenzène sulfonique, il ne se produit aucune dispersion. Il n'y a pas davantage de réaction que ce soit avec l'acide carboxylique, alcoyl- ou alcoylarylsulfonique.

Gradeff et al dans US-A 4 545 923 ont proposé un nouveau type de dioxyde cérique colloïdal à forte teneur en cérium, qui est susceptible de se disperser dans des liquides organiques, notamment des solvants organiques. Elle concerne également des compositions à forte teneur en cérium contenant ledit dioxyde cérique colloïdal dispersé dans un liquide organique. Conformément à l'invention, les dispersions à forte teneur en cérium sont de vraies dispersions comme cela est démontré par microscopie électronique en transmission. L'expression "dioxyde de cérium dispersé" utilisée dans la description et les revendications signifie que les particules de dioxyde cérique sont de dimensions colloïdales et que, par conséquent, elles se trouvent sous la forme d'une dispersion colloïdale dans des liquides organiques, mais ceci n'exclut pas la présence de dioxyde cérique en solution, en plus du dioxyde cérique dispersé sous forme colloïdale. L'examen au microscope électronique en transmission du dioxyde cérique hydraté avant traitement selon l'invention ne met pas en évidence de particules de dimensions colloïdales. La transformation dudit dioxyde cérique en particules de dimensions colloïdales se fait au cours du traitement.
La figure 1 de US-A 4 545 923 représente une photographie prise au microscope électronique en transmission qui montre l'état particulaire cristallin d'un dioxyde cérique typique avant traitement effectué selon le procédé dudit brevet.
La figure 2 de US-A 4 545 923 représente une photographie prise au microscope électronique en transmission qui montre la forme particulaire du dioxyde cérique de la figure 1 après traitement effectué selon le procédé dudit brevet.

Cette forme de dioxyde cérique colloïdal est obtenue à partir de dioxyde cérique préparé spécialement pour servir de produit de départ dans le procédé dudit brevet de telle sorte qu'il retienne par des associations physiques :
(1) - de 3 environ à 14 % environ de nitrate d'ammonium
(2) - au moins l'un des membres du groupe comprenant l'eau, le méthanol, l'acide acétique et les mélanges de deux ou trois quelconques de ces membres en une quantité allant d'environ 10 à environ 60 g par mole de CeO₂.

(1) et (2) sont essentiels et doivent obligatoirement être présents. On dénommera le produit obtenu "dioxyde cérique activé" ou "CeO₂ activé".

Il a été mis en évidence expérimentalement que le procédé dudit brevet peut être considéré comme une réaction physique adsorption-addition (par opposition à une réaction chimique substitution - élimination telle que la formation d'un sel) de l'acide organique, peut-être au niveau des interstices ou comme inclusion par chimisorption dans la structure du dioxyde cérique, qu'il soit cristallin ou non. Cette association physique se forme lors de la rupture de gros agglomérats de dioxyde cérique en cristallites ayant un diamètre d'environ 5 nm (50 Å), par chauffage du dioxyde cérique activé précité, en présence d'un acide organique solubilisant ayant de 10 à 40 atomes de carbone et d'un liquide organique approprié, à une température variant dans l'intervalle d'environ 60°C à environ 200°C pendant une durée suffisante, généralement de 1 heure à environ 24 heures, pour effectuer la désagrégation des agglomérats en cristallites de dimensions colloïdales et leur association avec l'acide solubilisant ; ensuite, on élimine l'eau, le méthanol ou l'acide acétique libéré, puis on élimine par filtration les sels qui se séparent lors du refroidissement.

Le complexe qui résulte de l'association physique CeO₂ - acide peut être isolé de telles solutions colloïdales sous forme de particules colloïdales solides. La microscopie électronique en transmission des solutions colloïdales met en évidence des cristallites de 5 nm (50 Å), parfaitement dispersées. Le complexe reste stable pendant un certain temps à condition qu'il soit conservé dans un récipient fermé. Si on le mélange avec un liquide organique approprié, on obtient aussitôt une dispersion colloïdale.

Toutefois, ce complexe associatif n'est pas dispersable dans l'alcool pour former une dispersion colloïdale et ne peut donc pas être utilisé où une dispersabilité dans l'alcool est requise. Une telle dispersion remplirait un besoin commercial spécifique.

Le procédé décrit dans US-A 4 545 923 consiste :
(1) à chauffer à une température comprise dans un intervalle allant d'environ 60°C à environ 200°C
   (a) du dioxyde cérique comprenant du nitrate d'ammonium en une quantité comprise entre environ 3 % et environ 14 % du poids du dioxyde cérique et un membre choisi dans le groupe formé par l'eau, le méthanol, l'acide acétique et leurs mélanges en une quantité d'au moins 10 g par mole de CeO₂ suffisante pour réagir avec
   (b) un acide organique ayant environ 10 à environ 40 atomes de carbone et
   (c) un liquide organique choisi dans le groupe formé par les hydrocarbures aliphatiques, cycloaliphatiques et aromatiques ; les éthers aliphatiques et cycloaliphatiques; les cétones aliphatiques et cycloaliphatiques
   formant ainsi une dispersion colloïdale dans le liquide organique du dioxyde cérique et de l'acide organique associé ;
(2) à éliminer l'eau, le méthanol, l'acide acétique libéré pendant le chauffage et à séparer toutes les particules solides non dissoutes.

Conformément à la présente invention, il a été trouvé que, si l'acide organique est un acide hydroxyphénylcarboxylique et le solvant organique un alcool aliphatique ou un mélange alcool-éther ou alcool-cétone, on obtient un complexe associatif dispersable dans l'alcool mais non dispersable dans les solvants hydrocarbonés.

La présente invention propose un procédé de préparation de dispersions colloïdales de complexes associatifs dispersables dans l'alcool comprenant du dioxyde cérique et un acide hydroxyphényl carboxylique ayant environ de 7 à environ 20 atomes de carbone dans le rapport molaire acide organique/CeO₂ d'au moins 1/6 qui consiste :
(1) à mélanger à une température comprise dans un intervalle allant de la température ambiante à environ 100°C
   (a) du dioxyde cérique comprenant du nitrate d'ammonium en une quantité comprise entre environ 3 % et environ 14 % du poids de dioxyde cérique et un membre choisi dans le groupe formé par l'eau, le méthanol, l'acide acétique et leurs mélanges en une quantité d'au moins 10 g par mole de CeO₂ suffisante pour réagir avec
   (b) un acide hydroxyphénylcarboxylique ayant environ de 7 à environ 20 atomes de carbone et
   (c) un alcool éventuellement porteur d'un groupement éther ou cétone
   formant ainsi une dispersion colloïdale dans l'alcool du dioxyde cérique et de l'acide organique associé ;
(2) à éliminer l'eau, le méthanol, l'acide acétique libéré pendant le chauffage et à séparer toutes les particules solides non dissoutes.

Comme dans US-A 4 545 923, le procédé de l'invention peut être considéré comme une réaction physique adsorption-addition (par opposition à une réaction chimique substitution-élimination telle que la formation d'un sel) de l'acide hydroxyphénylcarboxylique, peut être au niveau des interstices ou comme inclusion par chimisorption dans la structure du dioxyde cérique, qu'il soit cristallin ou non. Selon la présente invention, le complexe associatif est dispersable dans l'alcool, ce qui est dû à la présence de l'acide hydroxyphénylcarboxylique solubilisant ayant de 7 à 20 atomes de carbone, et d'un alcool, à une température comprise dans un intervalle allant de la température ambiante à environ 100°C pendant une durée suffisante généralement moins de 1 heure à environ 24 heures, pour effectuer la désagrégation des agglomérats en cristallites de dimensions colloïdales et leur association avec l'acide solubilisant et dispersion dans l'alcool ; puis élimination de l'eau, du méthanol et de l'acide acétique libéré, et filtration des sels qui se séparent lors du refroidissement. Avantageusement, l'alcool est un alcool aliphatique liquide ayant de 1 à environ 10 atomes de carbones, de préférence un alcool aliphatique inférieur.

Ces dispersions alcooliques sont composées de particules colloïdales dispersées. Le complexe dispersable dans l'alcool qui résulte de l'association physique CeO₂ et acide peut être isolé de telles dispersions colloïdales sous forme de particules solides colloïdales. La microscopie électronique en transmission des dispersions colloïdales met en évidence que les dispersions sont très uniformes et homogènes. Le complexe reste stable pendant un certain temps à condition qu'il soit conservé dans un récipient fermé. Si on le mélange avec un alcool approprié, on obtient aussitôt une dispersion colloïdale.

Le dioxyde cérique de départ peut être un dioxyde cérique anhydre ou un dioxyde cérique hydraté mais il est essentiel que le produit de départ du dioxyde cérique contienne de l'ordre de 3 à 14 % en poids de nitrate d'ammonium. On ne peut pas tout simplement mélanger ou ajouter le nitrate d'ammonium au dioxyde cérique. Il faut qu'il y ait une association physique étroite entre la nitrate d'ammonium et le dioxyde cérique ; il est possible que ce soit par inclusion du nitrate d'ammonium sous forme de molécule saline et/ou sous forme d'ions ammonium et nitrate dans la structure des agglomérats trouves au cours de la préparation du dioxyde cérique hydraté, selon un mode avantageux de l'invention on mélange le dioxyde cérique contenant le nitrate d'ammonium en présence d'eau ou d'alcool à température variant dans l'intervalle allant de la température ambiante à environ 100°C, puis on ajoute l'acide hydroxyphénylcarboxylique au mélange résultant". La deuxième condition exigée est la présence dans le systeme de la quantité précitee d'eau, de méthanol, d'acide acétique ou de leurs mélanges. Le dioxyde cérique est avantageusement sous forme d'une bouillie aqueuse ou d'une bouillie dans un alcool.

Le dioxyde cérique de départ convenant pour la préparation des produits de l'invention est commercialisé par la Société Rhône-Poulenc. On peut également le préparer par les procédés décrits dans les brevets, par exemple la demande de brevet française publiée sous le n° 2 482 075 qui décrit le traitement du nitrate céreux ou du carbonate céreux par de l'acide nitrique aqueux suivi par un traitement NH₄OH-H₂O₂. Aux fins de la présente invention, le dioxyde cérique qui est récupéré, par exemple par filtration, centrifugation ou autre technique de séparation, n'a pas besoin d'être lavé ou s'il est lavé, il ne l'est pas suffisamment pour éliminer le nitrate d'ammonium occlus ; par conséquent, il contient par association physique de l'ordre de 3 à 14 % de nitrate d'ammonium résiduel ainsi qu'un peu de nitrate de cérium. La quantité de nitrate peut varier en fonction des paramètres de procédé retenus pour la préparation, en fonction de la quantité des eaux-mères résiduelles ou de l'importance du lavage partiel lorsqu'il est effectué. Il va de soi que lorsque la base utilisée pour la précipitation est NH₄OH, les ions portés par le dioxyde cérique seront NH₄⁺ et NO₃⁻.

Le produit humide provenant directement du filtre contient donc une quantité variable d'eau. Dans le cas où la deuxième condition exigée est remplie par la présence d'eau, on peut noter qu'il faut au moins environ 10 g d'eau/mole de CeO₂ pour que le produit humide puisse être mis en oeuvre dans le cadre de l'invention. Normalement, la quantité d'eau retenue dans le dioxyde cérique hydraté qui vient d'être préparé est de l'ordre de 10 à 20 %. Il peut y avoir évidemment présence d'une plus grande quantité d'eau, mais c'est un inconvénient puisqu'il faudra l'éliminer plus tard au cours du procédé.

Il est surprenant de constater que si le méthanol peut se substituer à l'eau, les autres alcools inférieurs, tels que l'éthanol, restent sans effet et ne peuvent pas se substituer au méthanol.

De même, l'acide acétique est le seul acide que l'on puisse substituer à l'eau ou au méthanol ; on ne peut pas mettre en oeuvre l'acide organique qui est utilisé pour la préparation du complexe obtenu par association physique. L'acide acétique aussi bien que l'eau ou le méthanol a, de toute évidence, une fonction particulière dans le mécanisme qui n'est pas encore entièrement élucidé de désagrégation des agglomérats en CeO₂ de dimensions colloïdales suivie par l'addition de l'acide solubilisant.

La quantité d'eau, de méthanol, d'acide acétique ou de leurs mélanges est d'au moins 10 à environ 60 g/mole de CeO₂.

Il ne faut pas procéder à un séchage prolongé du dioxyde cérique a des températures élevées, par exemple 375°C ou plus, qui entraineraient la décomposition du nitrate d'ammonium : dans ce cas, la teneur du dioxyde cérique obtenu en NH₄NO₃ pourrait tomber en-dessous de la quantité minimum requise et ledit dioxyde cérique ne peut plus être utilisé dans le procédé de l'invention même après addition d'eau, de méthanol, d'acide ou même de nitrate d'ammonium libre.

Le milieu liquide organique utilisé dans le procédé de l'invention peut être un alcool aliphatique ou aromatique inerte ou un mélange d'entre eux qui soit liquide à la température réactionnelle choisie tel que par exemple, le méthanol, l'éthanol, le propanol, l'isopropanol, le butanol, l'isobutanol, l'alcool sec-butylique, l'alcool tert-butylique, le pentanol, l'isopentanol, l'hexanol, l'heptanol, l'octanol, l'ethyl-2 hexanol, le nonanol et le décanol, l'alcool benzylique, l'alcool α-méthylbenzylique.

Il n'est pas possible selon l'invention d'utiliser comme solvants, un hydrocarbure aliphatique ou aromatique, un éther, une cétone puisque la réaction avec l'acide hydroxyphénylcarboxylique n'a pas lieu dans un tel milieu. Toutefois, la réaction se produit avec des alcools porteurs d'un autre groupement fonctionnel, tel qu'un groupement éther ou cétone, en plus du groupement hydroxyle. On peut citer l'éthoxy-2 éthanol, le propoxy-3 propanol, l'ether méthylique du diéthylèneglycol, l'éther éthylique du diéthylèneglycol, etc...

Le choix de l'alcool ou solvant est fait en tenant compte de la solubilité de l'acide hydroxyphénylcarboxylique utilisé, de la température réactionnelle aussi bien que l'application finale de la dispersion colloïdale. Il est préférable dans certains cas de faire appel à un mélange de solvants alcooliques. La quantité de solvant alcoolique utilisée détermine évidemment la concentration finale. Les dispersions contenant jusqu'à environ 50 % de CeO₂ sont parfaitement fluides. Il est donc plus économique et plkus commode de préparer des dispersions plus concentrées qui pourront être diluées plus tard, lors de leur emploi. C'est pour cette raison que la quantité d'alcool n'est pas critique.

Un acide hydroxyphénylcarboxylique formant un complexe associatif soluble dans l'alcool répond à la formule (I) suivante : 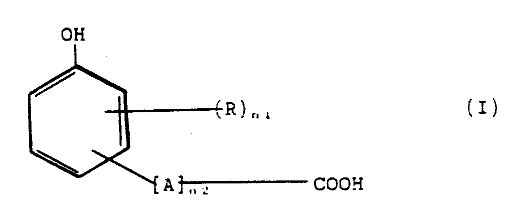
dans ladite formule (I)
- R représente un atome d'hydrogène ou un radical alkyle ayant une faible condensation en carbone de 1 à environ 4 atomes de carbone,
- A représente un lien valentiel ou un radical divalent hydrocarboné saturé ou insaturé ayant de 1 à environ 10 atomes de carbone,
- n1 est égal à 0, 1 ou 2
- n2 est égal à 0 ou 1

Le groupe A-COOH peut être en méta ou para par rapport à OH. De préférence, il est en position para du groupe OH.

A titre d'exemples de radicaux R, on peut citer le radical méthyle, éthyl, propyle, isopropyle, butyle, isobutyle, sec-butyle et tert-butyle.

A titre d'exemples de groupes A, on peut citer les radicaux divalents suivants : -CH₂-, -CH₂-CH₂-, -CH₂CH₂CH₂-, 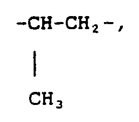 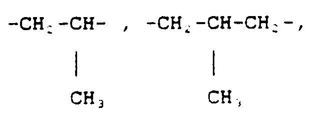
-CH₂ -CH₂ -CH₂ -CH₂-, 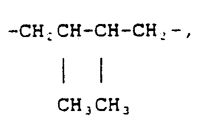
-(̵CH₂)̵₆, 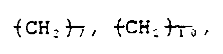
-CH=CH-, -CH=CH-CH₂-, -CH₂ -CH=CH-.

Comme exemples d'acides hydroxyphénylcarboxyliques convenant bien à l'invention, on peut faire appel à :
- l'acide p-hydroxyphénylacétique
- l'acide m-hydroxyphénylacétique
- l'acide p-hydroxyphénylpropionique
- l'acide m-hydroxyphénylpropionique
- l'acide p-hydroxyphénylbutyrique
- l'acide p-hydroxybenzoïque
- l'acide p-hydroxycinnamique
Le type d'acide organique solubilisant utilisé détermine le plus souvent la quantité maximum de CeO₂ qui puisse être dissoute.

L'acide organique est mis en oeuvre à raison d'au moins 0,15 mole par mole de CeO₂ étant donné que le complexe obtenu par association physique CeO₂-acide présente un rapport acide organique/CeO₂ d'au moins 1/6 comme le montre la composition de la forme solide isolée. On peut, certes, employer de plus petites quantités d'acide, mais on risque d'obtenir une dispersion incomplète du dioxyde cérique ou une dispersion relativement instable qui aura tendance à former un dépôt de CeO₂. On peut employer plus de 0,25 mole d'acide organique, mais ce n'est peut-être pas nécessaire. Ainsi la présente invention vise également un complexe associatif comprenant du dioxyde cérique et un acide hydroxyphénylcarboxylique ayant environ de 7 à environ 20 atomes de carbone dans le rapport molaire acide organique /CeO₂ d'au moins environ 1/6".

La présence d'eau, de méthanol ou d'acide acétique ou de leur mélange est indispensable durant la réaction mais l'on n'explique pas très bien leur rôle. Tout au moins, on peut dire qu'ils contribuent à l'expulsion des ions nitrate d'une manière telle qu'il en résulte une désagrégation des agglomérats de CeO₂ en particules de dimensions colloïdales. La surface hautement activée des cristallites adsorbe alors l'acide qui les rend dispersables dans l'alcool. Si l'on enlève du système, l'un des activateurs volatils essentiels, à savoir l'eau, le méthanol ou l'acide acétique, avant que les processus désirés aient eu lieu, il peut arriver que la réaction ne se produise pas du tout ou soit incomplète.

Les dioxydes cériques hydratés techniques du commerce contiennent d'autres terres rares comme impuretés. Dans certains cas, on peut souhaiter la présence de telles impuretés en raison d'effets de synergie bénéfiques qu'elles peuvent présenter. On peut également utiliser selon le procédé de l'invention des mélanges de dioxyde cérique contenant jusqu'à environ 10 % d'autres terres rares.

La durée totale du chauffage est de moins d'une heure à environ 24 heures et plus et pendant ce temps, on agite et chauffe (si on le désire) à une température comprise dans un intervalle allant de la température ambiante (le plus souvent comprise entre 15°C et 25°C) à 200°C environ.

En procédant à un chauffage préliminaire du dioxyde cérique de départ se présentant soit sous forme de bouillie aqueuse ou soit en mélange avec un alcool, à une température comprise dans un intervalle allant de 60°C environ à 200°C environ, pendant plusieurs heures suivi de l'addition de l'acide hydroxyphénylcarboxylique utilisé pour former le complexe obtenu par association physique, on peut obtenir de manière significative des vitesses de solubilisation plus rapides. L'examen au microscope électronique du produit chauffé a révélé qu'il ne s'est produit aucune diminution de la taille des particules de dioxyde cérique et par conséquent, on est amené à penser que le chauffage affaiblit, mais ne rompt pas les ponts entre les cristallites de nitrate d'ammonium et/ou d'ions NH₄⁺ et NO₃⁻. Il apparait que, si l'on procède à un traitement dans des conditions réactionnelles douces, le dioxyde cérique est réduit à des dimensions colloïdales par adsorption de l'acide hydroxyphénylcarboxylique, sur les particules de dioxyde cérique ce qui rend aussi, les particules colloïdales dispersables dans l'alcool

Par l'expression "dispersable dans l'alcool", on entend dispersables dans les alcools aliphatiques ou aromatiques. Toutefois, les solvants préférés sont les alcools aliphatiques inférieurs tels que, par exemple, le méthanol, l'éthanol, les propanols, les butanols, les pentanols et les hexanols. On pense que les dispersions colloïdales obtenues selon le procédé décrit contiennent l'acide solubilisant sous forme d'acide libre et non pas sous forme ionisée. Les produits à base de dioxyde de cérium décrits ci-dessus ne peuvent pas être considérés comme des savons de cérium étant donné que ces savons sont essentiellement des sels de cérium d'acides gras ionisés.

Les exemples qui suivent illustrent l'invention, sans pour autant la limiter.

### EXEMPLES 1 à 8

### 1°) Préparation de la dispersion colloïdale

Dans un réacteur à trois cols de 250 cm³ équipé d'un agitateur, d'un thermomètre et d'un condenseur, on introduit une solution d'acide p-hydroxyphénylacétique (9,42 g, 0,061 mole) dans 35 g d'éthoxy-2 éthanol. A cette solution, on additionne sous agitation 55,09 g de CeO₂,xH₂O contenant 63,03 % de cérium équivalent à 0,248 mole. Il se forme une bouillie marron clair qui se transforme en une solution marron foncé, en environ 40 minutes, à température ambiante. On chauffe le milieu réactionnel lentement jusqu'à 90°C, pour obtenir une complète dispersion, puis on laisse refroidir à température ambiante.

On additionne 12,50 g d'éthoxy-2 éthanol.

On place ensuite un appareil Dean and Stark et l'on élimine l'eau azéotropiquement (98°C) sous vide assuré par une pompe à huile.

On élimine au total 7,62 g d'eau.

On refroidit la solution à température ambiante et on filtre.

Par analyse des cendres, on détermine une teneur en cérium du filtrat de 34,04 %.

### 2°) Isolement sous forme solide d'un complexe CeO₂-acide dispersable dans l'alcool

On additionne goutte à goutte, 20 g du produit dispersable dans l'alcool préparé sous 1°, dans 40 g d'acétone. On filtre un précipité brunâtre, on le lave 3 fois avec 30 cm³ d'acétone et on le sèche sous vide pendant 24 heures.

L'analyse des cendres donne 65,86 % de cérium, ce qui révèle une composition comprenant 4 moles CeO₂ et une mole d'acide (théorie 66,5 %).

On effectue une série d'essais mettant en oeuvre les différents acides et solvants donnés dans le Tableau I, en suivant le même protocole opératoire, en utilisant le même dioxyde cérique activé et en mettant en oeuvre une quantité d'acide entre 0,022 et 0,25 mole par mole de CeO₂.

Dans tous les cas, on obtient une dispersion complète du dioxyde cérique et l'on trouve que le produit réactionnel est totalement dispersable dans les alcools courants.

**Tableau I**

| Exemple | Acide | Solvant | Dispersabilité | |
|---|---|---|---|---|
| | | | Méthanol/éthanol/isopropanol | AMSCO/heptane/toluène |
| 1 | acide p-hydroxyphényl acétique | éthoxy-2 éthanol | oui | non |
| 2 | acide p-hydroxy cinnamique | éthoxy-2 éthanol | oui | non |
| 3 | acide m-hydroxy cinnamique | éthoxy-2 éthanol | oui | non |
| 4 | acide p-hydroxy benzoique | éthoxy-2 éthanol | oui | non |
| 5 | acide m-hydroxy benzoique | éthoxy-2 éthanol | oui | non |
| 6 | acide p-hydroxyphényl acétique | méthanol | oui | non |
| 7 | acide p-hydroxyphényl acétique | octanol-2 | oui | non |
| 8 | acide p-hydroxyphényl acétique | alcool benzylique | oui | non |

### Exemple 9

On met en oeuvre la dispersion colloïdale de l'exemple 1.

On note qu'elle est complètement dispersable dans l'alcool benzylique.

## Revendications

1. Procédé de préparation de dispersions colloïdales de complexes associatifs dispersables dans l'alcool comprenant du dioxyde cérique et un acide hydroxyphénylcarboxylique ayant environ de 7 à environ 20 atomes de carbone dans le rapport molaire acide organique/CeO₂ d'au moins 1/6 caractérisé par le fait qu'il consiste :
(1) à mélanger à une température comprise dans un intervalle allant de la température ambiante à environ 100°C
(a) du dioxyde cérique comprenant du nitrate d'ammonium en une quantité comprise entre environ 3 % et environ 14 % du poids de dioxyde cérique et un membre choisi dans le groupe formé par l'eau, le méthanol, l'acide acétique et leurs mélanges en une quantité d'au moins 10 g par mole de CeO₂ suffisante pour réagir avec
(b) un acide hydroxyphénylcarboxylique ayant environ de 7 à environ 20 atomes de carbone et
(c) un alcool éventuellement porteur d'un groupement éther ou cétone
formant ainsi une dispersion colloïdale dans l'alcool du dioxyde cérique et de l'acide organique associé ;
(2) à éliminer l'eau, le méthanol, l'acide acétique libéré pendant le chauffage et à séparer toutes les particules solides non dissoutes.

2. Procédé selon la revendication 1 caractérisé en ce que l'on utilise l'eau, le méthanol ou l'acide acétique ou un mélange d'eau et de méthanol, d'eau et d'acide acétique ou d'eau, de méthanol et d'acide acétique.

3. Procédé selon l'une des revendications 1 et 2 caractérisé par le fait que l'alcool est un alcool aliphatique liquide ayant de un à environ 10 atomes de carbone ou un alcool aliphatique-éther.

4. Procédé selon l'une des revendications 1 et 2 caractérisé par le fait que l'alcool est un alcool aliphatique inférieur.

5. Procédé selon l'une des revendications 1 à 4 caractérisé par le fait que l'acide hydroxyphénylcarboxylique répond à la formule (I) suivante : 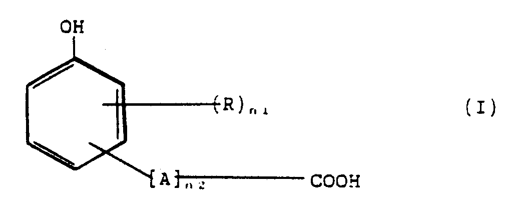 dans ladite formule (I)
- R représente un atome d'hydrogène ou un radical alkyle ayant une faible condensation en carbone de 1 à environ 4 atomes de carbone,
- A représente un lien valentiel ou un radical divalent hydrocarboné saturé ou insaturé ayant de 1 à environ 10 atomes de carbone,
- n1 est égal à 0, 1 ou 2
- n2 est égal à 0 ou 1
et le groupe A-COOH est en méta ou para rapport à OH.

6. Procédé selon la revendication 5 caractérisé par le fait que l'acide hydroxyphénylcarboxylique est un acide p-hydroxybenzoique, p-hydroxyphénylacétique, p-hydroxycinnamique.

7. Procédé selon la revendication 6 caractérisé par le fait que l'acide hydroxyphénylcarboxylique est l'acide p-hydroxyphénylacétique.

8. Procédé selon la revendication 1 caractérisé par le fait que la quantité d'eau, de méthanol ou d'acide acétique varie dans l'intervalle d'environ 10 à environ 60 g par mole de CeO₂.

9. Procédé selon l'une des revendications 1 à 8 caractérisé par le fait que la quantité d'acide hydroxyphénylcarboxylique est d'au moins 0,15 mole par mole de CeO₂.

10. Procédé selon la revendication 9 caractérisé par le fait que ladite quantité est d'environ 0,25 mole par mole de CeO₂.

11. Procédé selon la revendication 1 caractérisé par le fait que l'on mélange le dioxyde cérique contenant le nitrate d'ammonium en présence d'eau ou d'alcool à une température variant dans l'intervalle allant de la température ambiante à environ 100°C et puis on ajoute l'acide hydroxyphénylcarboxylique au mélange résultant.

12. Procédé selon la revendication 98 caractérisé par le fait que le dioxyde cérique est sous la forme d'une bouillie aqueuse ou d'une bouillie dans un alcool.

13. Procédé selon l'une des revendications 1 à 12 caractérisé par le fait que la température est comprise entre 60°C environ et 200°C environ.

14. Complexe associatif comprenant du dioxyde cérique et un acide hydroxyphénylcarboxylique ayant environ de 7 à environ 20 atomes de carbone dans le rapport molaire acide organique/CeO₂ d'au moins environ 1/6.

15. Complexe selon la revendication 14 caractérisé par le fait que ledit rapport molaire est d'environ 1/4.

16. Complexe selon la revendication 14 caractérisé par le fait que l'acide hydroxyphénylcarboxylique répond à la formule (I) suivante : 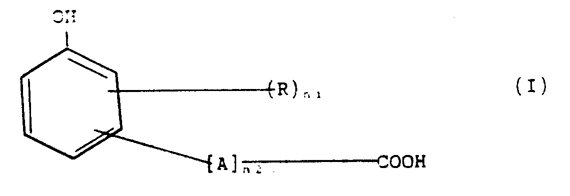 dans ladite formule (I)
- R représente un atome d'hydrogène ou un radical alkyle ayant une faible condensation en carbone de 1 à environ 4 atomes de carbone,
- A représente un lien valentiel ou un radical divalent hydrocarboné saturé ou insaturé ayant de 1 à environ 10 atomes de carbone,
- n1 est égal à 0, 1 ou 2
- n2 est égal à 0 ou 1
et le groupe A-COOH est en méta ou para par rapport à OH.

17. Complexe selon la revendication 16 caractérisé par le fait que l'acide hydroxyphénylcarboxylique est un acide p-hydroxybenzoique, p-hydroxyphénylacétique, p-hydroxycinnamique.

18. Complexe selon la revendication 17 caractérisé par le fait que l'acide hydroxyphénylcarboxylique est l'acide p-hydroxyphénylacétique.

19. Dispersion colloïdale du complexe associatif décrit dans l'une des revendications 14 à 18 dans un alcool aliphatique ou aromatique.

20. Dispersion colloïdale selon la revendication 19 caractérisée par le fait que l'alcool est un alcool aliphatique inférieur.

## Claims

1. Process for the preparation of colloidal dispersions of alcohol-dispersible associative complexes comprising ceric dioxide and a hydroxyphenylcarboxylic acid having approximately from 7 to approximately 20 carbon atoms in the organic acid/CeO₂ molar ratio of at least 1/6, characterized in that it consists:
(1) in mixing, at a temperature within a range from room temperature to approximately 100°C,
(a) ceric dioxide comprising ammonium nitrate in an amount of between approximately 3% and approximately 14% of the weight of ceric dioxide and a member chosen from the group formed by water, methanol, acetic acid and their mixtures in an amount of at least 10 g per mole of CeO₂ sufficient to react with
(b) a hydroxyphenylcarboxylic acid having approximately from 7 to approximately 20 carbon atoms and
(c) an alcohol optionally carrying an ether or ketone group
thus forming a colloidal dispersion, in the alcohol, of ceric dioxide and the associated organic acid;
(2) in removing the water, methanol or acetic acid released during the heating and in separating all the undissolved solid particles.

2. Process according to claim 1, characterized in that water, methanol or acetic acid or a mixture of water and methanol, water and acetic acid or water, methanol and acetic acid is used.

3. Process according to either of claims 1 and 2, characterized in that the alcohol is a liquid aliphatic alcohol having from one to approximately 10 carbon atoms or an ether-aliphatic alcohol.

4. Process according to either of claims 1 and 2, characterized in that the alcohol is a lower aliphatic alcohol.

5. Process according to one of claims 1 to 4, characterized in that the hydroxyphenylcarboxylic acid corresponds to the following formula (I): 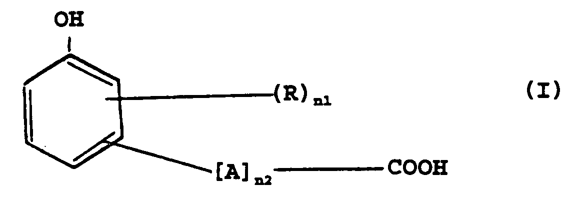 in the said formula (I)
- R represents a hydrogen atom or an alkyl radical having a low carbon condensation of 1 to approximately 4 carbon atoms,
- A represents a valency bond or a saturated or unsaturated divalent hydrocarbon radical having from 1 to approximately 10 carbon atoms,
- n1 is equal to 0, 1 or 2
- n2 is equal to 0 or 1
and the group A-COOH is meta or para with respect to OH.

6. Process according to claim 5, characterized in that the hydroxyphenylcarboxylic acid is a p-hydroxybenzoic, p-hydroxyphenylacetic or p-hydroxycinnamic acid.

7. Process according to claim 6, characterized in that the hydroxyphenylcarboxylic acid is p-hydroxyphenylacetic acid.

8. Process according to claim 1, characterized in that the amount of water, methanol or acetic acid varies within the range from approximately 10 to approximately 60 g per mole of CeO₂.

9. Process according to one of claims 1 to 8, characterized in that the amount of hydroxyphenylcarboxylic acid is at least 0.15 mol per mole of CeO₂.

10. Process according to claim 9, characterized in that the said amount is approximately 0.25 mol per mole of CeO₂.

11. Process according to claim 1, characterized in that ceric dioxide containing ammonium nitrate is mixed in the presence of water or alcohol at a temperature varying in the range from room temperature to approximately 100°C and then the hydroxyphenylcarboxylic acid is added to the resulting mixture.

12. Process according to claim 9, characterized in that the ceric dioxide is in the form of an aqueous slurry or of a slurry in an alcohol.

13. Process according to one of claims 1 to 12, characterized in that the temperature is between approximately 60°C and approximately 200°C.

14. Associative complex comprising ceric dioxide and a hydroxyphenylcarboxylic acid having approximately from 7 to approximately 20 carbon atoms in the organic acid/CeO₂ molar ratio of at least approximately 1/6.

15. Complex according to claim 14, characterized in that the said molar ratio is approximately 1/4.

16. Complex according to claim 14, characterized in that the hydroxyphenylcarboxylic acid corresponds to the following formula (I): 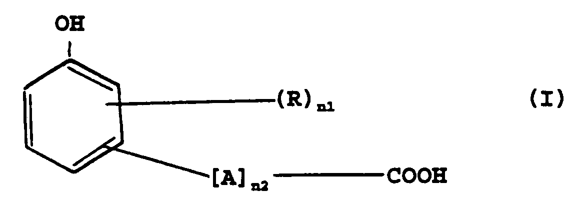 in the said formula (I)
- R represents a hydrogen atom or an alkyl radical having a low carbon condensation of 1 to approximately 4 carbon atoms,
- A represents a valency bond or a saturated or unsaturated divalent hydrocarbon radical having from 1 to approximately 10 carbon atoms,
- n1 is equal to 0, 1 or 2
- n2 is equal to 0 or 1
and the group A-COOH is meta or para with respect to OH.

17. Complex according to claim 16, characterized in that the hydroxyphenylcarboxylic acid is a p-hydroxybenzoic, p-hydroxyphenylacetic or p-hydroxycinnamic acid.

18. Complex according to claim 17, characterized in that the hydroxyphenylcarboxylic acid is p-hydroxyphenylacetic acid.

19. Colloidal dispersion of the associative complex disclosed in one of claims 14 to 18 in an aliphatic or aromatic alcohol.

20. Colloidal dispersion according to claim 19, characterized in that the alcohol is a lower aliphatic alcohol.

## Patentansprüche

1. Verfahren zur Herstellung von kolloidalen Dispersionen dispergierbarer, assoziativer Komplexe, die Cerdioxid und eine Hydroxyphenylcarbonsäure mit etwa 7 bis etwa 20 Kohlenstoffatomen in einem molaren Verhältnis organische Säure/CeO₂ von mindestens 1/6 umfassen, dadurch gekennzeichnet, daß es darin besteht:
(1) bei einer Temperatur, die im Intervall zwischen der Umgebungstemperatur und etwa 100 °C liegt, zu mischen
(a) Cerdioxid, das Ammoniumnitrat in einer Menge zwischen etwa 3 % und etwa 14 % des Gewichtes des Cerdioxids, und ein Bestandteil aus der durch Wasser, Methanol, Essigsäure und ihren Mischungen gebildeten Gruppe umfaßt, in einer Menge von mindestens 10 g pro Mol CeO₂, die ausreichend ist, um zu reagieren mit
(b) einer Hydroxyphenylcarbonsäure mit etwa 7 bis etwa 20 Kohlenstoffatomen und
(c) einem Alkohol, der gegebenenfalls eine Ethergruppe oder Ketongruppe trägt,
um auf diese Weise eine kolloidale Dispersion von Cerdioxid und der assoziierten organischen Säure in Alkohol zu bilden;
(2) das während des Erhitzens freiwerdende Wasser, Methanol und die Essigsäure zu entfernen und alle festen, nicht gelösten Teilchen abzutrennen.

2. Verfahren nach Anspruch 1, dadurch gekennzeichnet, daß man Wasser, Methanol oder Essigsäure oder eine Mischung von Wasser und Methanol, Wasser und Essigsäure oder Wasser, Methanol und Essigsäure verwendet.

3. Verfahren nach einem der Ansprüche 1 und 2, dadurch gekennzeichnet, daß der Alkohol ein flüssiger aliphatischer Alkohol mit 1 bis etwa 10 Kohlenstoffatomen oder ein aliphatischer Etheralkohol ist.

4. Verfahren nach einen der Ansprüche 1 und 2, dadurch gekennzeichnet, daß der Alkohol ein niederer aliphatischer Alkohol ist.

5. Verfahren nach einem der Ansprüche 1 bis 4, dadurch gekennzeichnet, daß die Hydroxyphenylcarbonsäure der folgenden Formel (I) entspricht: 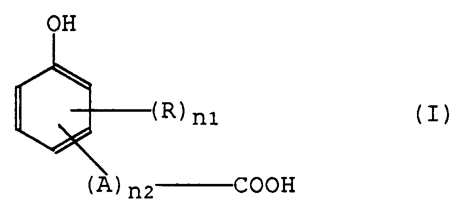 in der
- R ein Wasserstoffatom oder einen Alkylrest mit einer geringen Kondensation an Kohlenstoff von 1 bis etwa 4 Kohlenstoffatomen darstellt,
- A eine Valenzbindung oder einen gesättigten oder ungesättigten, divalenten Kohlenwasserstoffrest mit 1 bis etwa 10 Kohlenstoffatomen bedeutet,
- n1 gleich 0, 1 oder 2 ist,
- n2 gleich 0 oder 1 ist,
und die Gruppe A-COOH in meta- oder para-Stellung zu OH steht.

6. Verfahren nach Anspruch 5, dadurch gekennzeichnet, daß die Hydroxyphenylcarbonsäure eine p-Hydroxybenzoesäure, p-Hydroxyphenylessigsäure oder p-Hydroxyzimtsäure ist.

7. Verfahren nach Anspruch 6, dadurch gekennzeichnet, daß die Hydroxyphenylcarbonsäure die p-Hydroxyphenylessigsäure ist.

8. Verfahren nach Anspruch 1, dadurch gekennzeichnet, daß die Menge an Wasser, Methanol oder Essigsäure im Intervall von etwa 10 g bis etwa 60 g pro Mol CeO₂ schwankt.

9. Verfahren nach einem der Ansprüche 1 bis 8, dadurch gekennzeichnet, daß die Menge von Hydroxyphenylcarbonsäure mindestens 0,15 Mol pro Mol CeO₂ beträgt.

10. Verfahren nach Anspruch 9, dadurch gekennzeichnet, daß die genannte Menge etwa 0,25 Mol pro Mol CeO₂ beträgt.

11. Verfahren nach Anspruch 1, dadurch gekennzeichnet, daß man das Cerdioxid, das Ammoniumnitrat enthält, in Anwesenheit von Wasser oder Alkohol bei einer Temperatur, die im Intervall von der Umgebungstemperatur bis 100 °C liegt, vermischt und anschließend die Hydroxyphenylcarbonsäure zu der resultierenden Mischung gibt.

12. Verfahren nach Anspruch 9, dadurch gekennzeichnet, daß das Cerdioxid in Form einer wäßrigen Aufschlämmung oder in Form einer Aufschlämmung in einem Alkohol vorliegt.

13. Verfahren nach einem der Ansprüche 1 bis 12, dadurch gekennzeichnet, daß die Temperatur zwischen etwa 60 °C und etwa 200 °C liegt.

14. Assoziativer Komplex, umfassend Cerdioxid und eine Hydroxyphenylcarbonsäure mit etwa 7 bis etwa 20 Kohlenstoffatomen im molaren Verhältnis organische Säure/CeO₂ von mindestens etwa 1/6.

15. Komplex nach Anspruch 14, dadurch gekennzeichnet, daß das molare Verhältnis etwa 1/4 beträgt.

16. Komplex nach Anspruch 14, dadurch gekennzeichnet, daß die Hydroxyphenylcarbonsäure der folgenden Formel (I) entspricht: 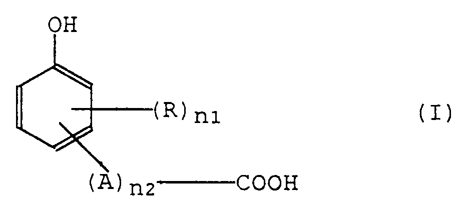 in der
- R ein Wasserstoffatom oder einen Alkylrest mit einer geringen Kondensation an Kohlenstoff von 1 bis etwa 4 Kohlenstoffatomen darstellt,
- A eine Valenzbindung oder einen gesättigten oder ungesättigten, divalenten Kohlenwasserstoffrest mit 1 bis etwa 10 Kohlenstoffatomen bedeutet,
- n1 gleich 0, 1 oder 2 ist,
- n2 gleich 0 oder 1 ist,
und die Gruppe A-COOH in meta- oder para-Stellung zu OH steht.

17. Komplex nach Anspruch 16, dadurch gekennzeichnet, daß die Hydroxyphenylcarbonsäure eine p-Hydroxybenzoesäure, p-Hydroxyphenylessigsäure oder p-Hydroxyzimtsäure ist.

18. Komplex nach Anspruch 17, dadurch gekennzeichnet, daß die Hydroxyphenylcarbonsäure die p-Hydroxyphenylessigsäure ist.

19. Kolloidale Dispersion des in einem der Ansprüche 14 bis 18 beschriebenen assoziativen Komplexes in einem aliphatischen oder aromatischen Alkohol.

20. Kolloidale Dispersion nach Anspruch 19, dadurch gekennzeichnet, daß der Alkohol ein niederer aliphatischer Alkohol ist.
